# EUROPEAN PATENT APPLICATION

(11) **EP 4 818 195 A1**
(43) Date of publication of application: **07.10.2026**
(21) Application number: 24862130.2
(22) Date of filing: 08.10.2024
(51) Int. Cl.: C07K 14/015, C12N 15/864, A61K 48/00, A01K 67/0275, A61P 27/02

(54) **CAPSID PROTEIN MUTANTS CAPABLE OF INCREASING ABILITY OF AAVS TO INFECT OCULAR TISSUE AND USE THEREOF**

(30) Priority: 16.01.2024 CN 202410056939; 16.01.2024 CN 202410062756; 21.06.2024 CN 202410804466; 21.06.2024 CN 202410804468; 25.07.2024 CN 202411002404; 18.09.2024 CN 202411302847
(71) Applicant: Guangzhou Yimagene Technology Co., Ltd., Guangzhou, Guangdong 510663 (CN)
(72) Inventor: YUAN, Li, Guangzhou, Guangdong 510663 (CN); ZHANG, Qian, Guangzhou, Guangdong 510663 (CN); REN, Sheng, Guangzhou, Guangdong 510663 (CN); HUO, Kugeng, Guangzhou, Guangdong 510663 (CN); GAO, Cui, Guangzhou, Guangdong 510663 (CN); LIU, Huaqing, Guangzhou, Guangdong 510663 (CN); MENG, Shan, Guangzhou, Guangdong 510663 (CN); LIU, Lei, Guangzhou, Guangdong 510663 (CN); WU, Chenguang, Guangzhou, Guangdong 510663 (CN); ZHENG, Qin, Guangzhou, Guangdong 510663 (CN)
(74) Representative: Fish & Richardson P.C.
(86) International application number: PCT/CN2024/123378
(87) International publication number: WO 2025/051292

(57) **Abstract**

Capsid protein mutants capable of increasing the ability of AAVs to infect ocular tissue and the use thereof. A Cap mutant is obtained by replacing an amino acid sequence of an AAV capsid protein which is homologous to amino acids at positions D561 to R588 of a wild-type AAV2 capsid protein with one of the polypeptides as shown in SEQ ID NOs. 6-39. Some Cap mutants can infect a mouse retina from the RGC layer, cone and rod cells of INL and ONL, corneal limbus regions, trabecular meshwork, RPE and a small amount of RPE cells by administration via intravitreous injection, can solve the problem that it is difficult for the conventional serotype AAV to infect the cells in the posterior layer of the retina due to insufficient penetrability, and is expected to become a potential carrier for the treatment of glaucoma. Experimental data show that the abilities of some Cap mutants to infect and penetrate the retina of non-human primates are optimized, thereby providing a new tool for developing gene therapy preparations for ophthalmic diseases or constructing animal models related to ophthalmic diseases.

## Description

### Technical Field

The present invention belongs to the field of biotechnology, and specifically relates to variant AAV capsid polypeptides capable of increasing the ability of adeno-associated viruses (AAVs) to infect ocular tissue and uses thereof. The present invention claims priority from the following Chinese patent applications:

| No. | Application No. | Filing Date |
|---|---|---|
| 1 | CN202410056939.3 | January 16, 2024 |
| 2 | CN202410062756.2 | January 16, 2024 |
| 3 | CN202410804466.0 | June 21, 2024 |
| 4 | CN202410804468.X | June 21, 2024 |
| 5 | CN202411002404.4 | July 25, 2024 |
| 6 | CN202411302847.5 | September 18, 2024 |

The entire contents of the above priority documents are incorporated herein by reference.

### Background Art

Adeno-associated virus (AAV) has a genome comprising approximately 4.7 kb of single-stranded DNA, contained within a 20 nm diameter icosahedral non-enveloped viral capsid, which can be divided into three functional regions: two open reading frames (Rep gene, Cap gene) and inverted terminal repeats (ITRs). Among them, the open reading frame of the Cap gene encodes three capsid polypeptides VP1, VP2 and VP3, with molecular weights of 87, 73, and 61 kDa, respectively. These are the AAV capsid polypeptides required for assembly into intact viruses and play important roles in viral integration, replication and assembly.

AAV is a delivery vector widely used in gene therapy. Its principle is to replace the sequence between the ITRs of the AAV genome with a target gene sequence through genetic engineering methods, and deliver it to target cells through cellular infection to achieve the purpose of gene therapy. Based on the characteristics of recombinant AAV such as safety, high efficiency, stability, persistence, specificity, and low integration, AAV has become one of the main delivery means in the field of gene therapy.

However, AAV still faces multiple challenges: first, the production process is complex, costly, and capacity is limited; second, high-dose administration may exhibit neurotoxicity and hepatotoxicity, causing safety issues; third, the infection ability for some tissue cells is weak, such as intravitreal administration being difficult to infect retinal cells in the posterior layer, and intravenous administration being difficult to infect central nervous system cells. Modifying and screening the AAV capsid polypeptide to improve its targeting and transduction efficiency for specific tissues, in order to achieve lower and safer dosing, is an effective way to solve this problem.

The eye has certain immune privilege due to the presence of the blood-eye barrier, and local administration is relatively safe. Moreover, fundus diseases are mostly monogenic genetic diseases, so ophthalmology has become a hot field for gene therapy. The commonly used injection methods in clinical practice are intravitreal injection and subretinal injection. Subretinal injection has good infection effects, especially for the RPE layer and outer nuclear layer, but this method is highly invasive, easily causes retinal detachment, and may lead to photoreceptor degeneration, visual function damage, and glial cell proliferation. Intravitreal injection has lower surgical risk, but conventional serotypes such as AAV2 and AAV5 can only infect the RGC layer, with poor infection effects on cone cells, rod cells and RPE. Therefore, developing AAV mutants that can improve whole-eye expression ability, or can penetrate the RGC layer and inner nuclear layer to infect outer nuclear layer cells and RPE cells, is of great significance for solving the problem of delivery vectors for ophthalmic gene therapy.

Previous studies have shown that partial amino acid substitution at positions D561 to R588 of the AAV2 capsid polypeptide Cap, or insertion of a polypeptide fragment of about 10 amino acids at positions 587 and 588, can improve its infection ability for various ocular tissues.

### Technical Problems

The prior art has made substantial modifications to the AAV capsid polypeptide, mainly targeting the infection ability of the whole eye, various retinal cell layers, and corneal limbus regions, and further improvements are needed.

### Technical Solution

An object of the present invention is to overcome at least one deficiency of the prior art, and to provide variant AAV capsid polypeptides capable of increasing the ability of AAV to infect ocular tissue and uses thereof.

The technical solutions adopted by the present invention are: In a first aspect, the present invention provides: a variant AAV capsid polypeptide capable of increasing the ability of an AAV to infect ocular tissue, obtained by deleting an amino acid sequence of an AAV capsid polypeptide which is homologous to amino acids at positions D561 to R588 of a wild-type AAV2 capsid polypeptide, and inserting one of the polypeptides shown in SEQ ID NOs: 6-39 at the deletion site.

In some embodiments, the AAV capsid polypeptide is a capsid polypeptide of AAV2 or an AAV2 variant. In particular, the AAV2 is a wild-type AAV2.

The amino acid sequence homologous to amino acids at positions D561 to R588 of a wild-type AAV2 capsid polypeptide can be determined based on existing methods, such as by comparative analysis of amino acid sequences of AAV capsid polypeptides of different serotypes.

Based on the analysis of the insertion position of the amino acid sequence, the amino acids inserted into the above AAV variant AAV capsid polypeptides will be displayed on the formed VP1, VP2 and VP3 proteins.

In a second aspect, the present invention provides: a gene encoding the AAV variant AAV capsid polypeptide according to the first aspect of the present invention.

In some embodiments, the gene is codon-optimized according to different expression systems.

In a third aspect, the present invention provides: an expression vector, which expresses the AAV variant AAV capsid polypeptide according to the first aspect of the present invention, or contains the gene according to the second aspect of the present invention.

In some embodiments, the expression vector is selected from plasmids and viral vectors. The expression vector can express the AAV variant AAV capsid polypeptide according to the first aspect of the present invention alone, and/or work together with other auxiliary expression vectors to ultimately form a self-assembling AAV capsid expression system, achieving packaging of the target nucleic acid molecule.

In some embodiments, the viral vector is a recombinant AAV vector obtained by insertion or replacement modification of an AAV vector.

In some embodiments, the AAV is selected from any one of AAV1, AAV2, AAV3, AAV4, AAV5, AAV6, AAV7, AAV8, AAV9, AAV10 and variants thereof.

In some embodiments, the expression vector further comprises a nucleic acid molecule encoding a functional gene product.

In some embodiments, the functional gene product is a factor acting on ocular cells.

In a fourth aspect, the present invention provides: a recombinant AAV, wherein the AAV capsid polypeptide thereof is as described in the first aspect of the present invention, or is obtained by cleaving the AAV capsid polypeptide described in the first aspect of the present invention.

In some embodiments, the AAV is selected from any one of AAV1, AAV2, AAV3, AAV4, AAV5, AAV6, AAV7, AAV8, AAV9, AAV10 and variants thereof.

In a fifth aspect, the present invention provides: an AAV particle, comprising a capsid and a gene packaged within the capsid, wherein the capsid comprises the AAV capsid polypeptide according to the first aspect of the present invention, or the capsid is assembled from capsid polypeptides VP1, VP2 and VP3 formed by the variant AAV capsid polypeptide according to the first aspect of the present invention, or the capsid is assembled from capsid polypeptides VP1, VP2 and VP3 encoded and expressed by the gene according to the first aspect of the present invention.

In some embodiments, depending on the application, the gene packaged within the capsid encodes a factor acting on ocular cells.

In a sixth aspect, the present invention provides: a composition, comprising the expression vector according to the third aspect of the present invention and a pharmaceutically acceptable carrier, or the recombinant AAV according to the fourth aspect of the present invention, or the AAV particle according to the fifth aspect of the present invention.

In a seventh aspect, the present invention provides: use of the composition according to the sixth aspect of the present invention, wherein the use includes but is not limited to: 1) preparing a gene therapy preparation or transgene preparation targeting ocular tissue; 2) constructing a transgenic animal targeting ocular tissue; and 3) preparing a drug or transgene vector targeting ocular tissue.

In an eighth aspect, the present invention provides: a gene therapy method for treating an ocular disease, comprising:
**S1.** detecting a patient to confirm that the patient suffers from an ocular disease;
**S2.** administering a therapeutically effective amount of a gene therapy preparation to the patient, wherein the gene therapy preparation is the recombinant AAV according to the fourth aspect of the present invention, and/or the AAV particle according to the fifth aspect of the present invention, and the nucleic acid molecule packaged in the recombinant AAV and the AAV particle expresses a factor capable of treating the ocular disease.

### Beneficial Effects

The variant AAV capsid polypeptides of some embodiments of the present invention can effectively improve the ability of AAV to infect ocular tissue. The 16 mutants AAV2-ROD-01, AAV2-ROD-02, AAV2-ROD-03, AAV2-ROD-05, AAV2-ROD-06, AAV2-ROD-07, AAV2-ROD-08, AAV2-ROD-09, AAV2-ROD-10, AAV2-ROD-11, AAV2-ROD-12, AAV2-ROD-15, AAV2-ROD-16, AAV2-ROD-17, AAV2-ROD-18, and AAV2-ROD-19 can infect cone and rod cells in the mouse retina from the retinal ganglion cell (RGC) layer, inner nuclear layer (INL) and outer nuclear layer (ONL), as well as a small amount of RPE cells, after intravitreal injection administration, which can solve the problem that conventional serotypes have insufficient penetration ability to infect retinal cells in the posterior layer. The mutants AAV2-EYE-08, AAV2-EYE-09, AAV2-EYE-10, AAV2-EYE-11, AAV2-EYE-12, AAV2-EYE-13, AAV2-EYE-14, AAV2-EYE-15, AAV2-EYE-16, AAV2-EYE-17, AAV2-EYE-18, AAV2-EYE-19, AAV2-EYE-20, AAV2-EYE-21, and AAV2-EYE-22 can improve the infection ability of the whole eye, with a wide infection range and high overall expression level. The mutant AAV2-A2HRE15 can infect the corneal limbus region, trabecular meshwork and RPE after intravitreal injection, and is expected to become a potential vector for treating glaucoma. AAV2-NE01 and AAV2-NE02 have optimized infection ability and penetration ability for non-human primate (NHP) retina compared with AAV2-WT, and can better infect various cell layers of NHP retina from the ganglion cell layer (GCL), inner nuclear layer (INL) to outer nuclear layer (ONL), as well as the corneal limbus region. The above variants solve the problem of AAV2 infection ability for multiple ocular tissues from different aspects, providing new tools for developing gene therapy preparations for ophthalmic diseases or constructing animal models related to ophthalmic diseases.

### Description of Drawings

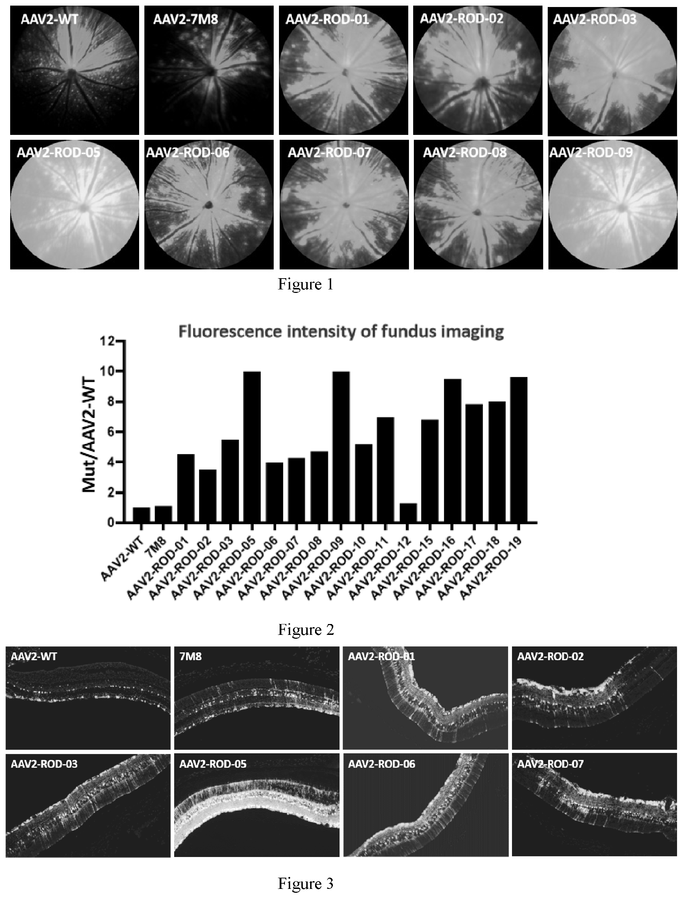

Figures 1-2 are EGFP expression effect diagrams of mouse eyes infected for 3 weeks with wild-type AAV2, AAV2-7M8 and AAV2-ROD-01 to AAV2-ROD-19 mutant viruses prepared by the scheme of the present invention in the test examples of the present invention. Figure 1 is a fundus fluorescence imaging diagram, and Figure 2 is a quantitative statistical diagram of fluorescence intensity of fundus fluorescence imaging.

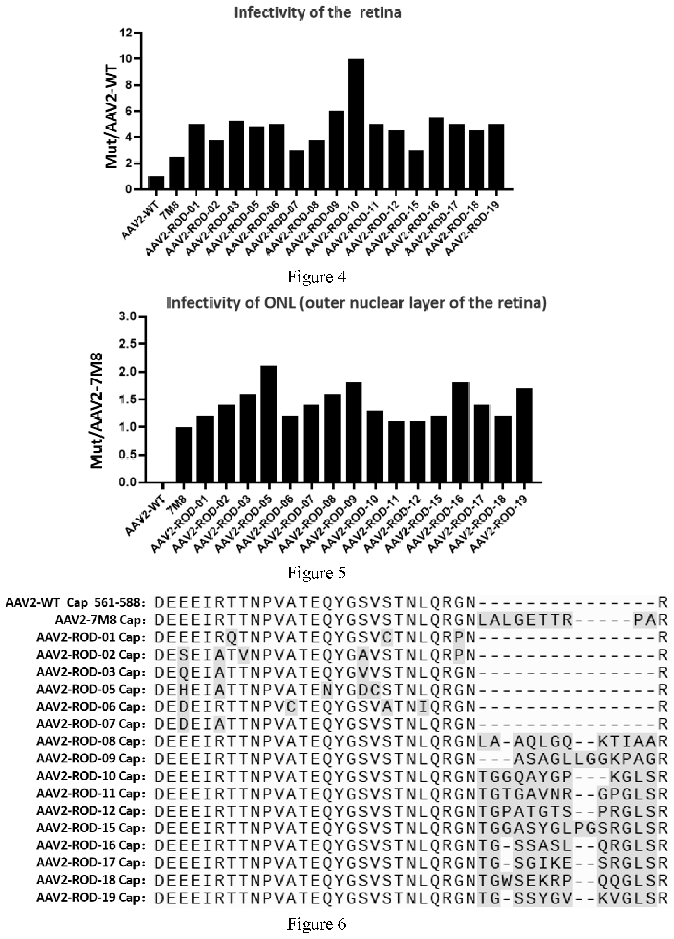

Figures 3-5 are retinal infection effect diagrams of mouse ocular tissue infected for 3 weeks with wild-type AAV2, AAV2-7M8 and AAV2 mutant viruses prepared by the scheme of the present invention in the test examples of the present invention. Figure 3 is a fluorescence photograph of retinal frozen sections, Figure 4 is a quantitative statistical diagram of fluorescence intensity in the whole retinal region, and Figure 5 is a quantitative statistical diagram of fluorescence intensity of photoreceptor cells in the posterior layer of the retina.
Figure 6 is a Cap 561-588 amino acid sequence alignment diagram of wild-type AAV2, AAV2-7M8 and AAV2-ROD-01 to AAV2-ROD-19 mutant sequences prepared by the scheme of the present invention in the test examples of the present invention.

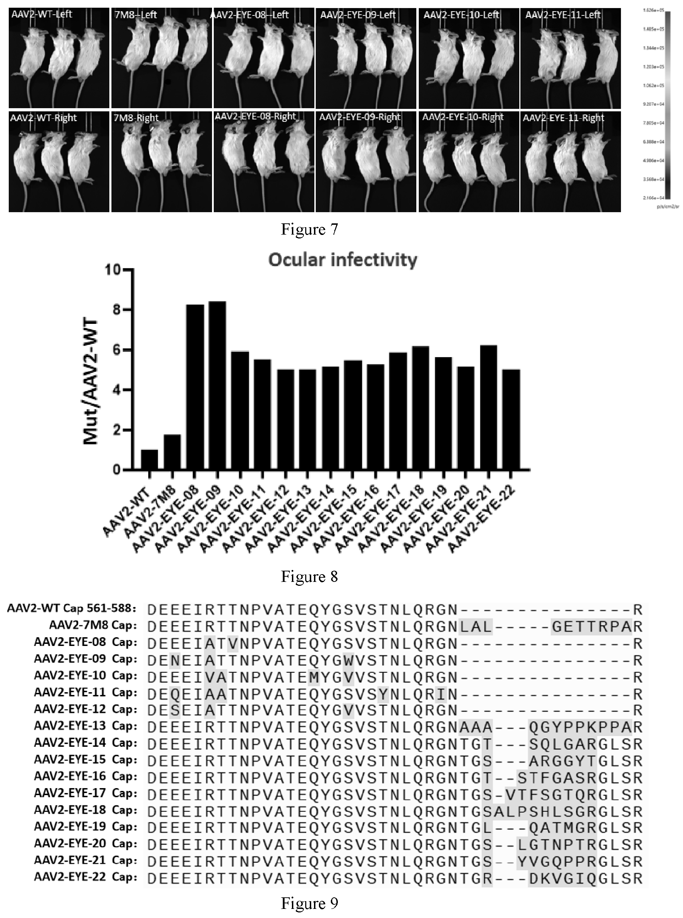

Figures 7-8 are Luciferase expression effect diagrams of mouse eyes infected for 3 weeks with wild-type AAV2, AAV2-7M8 and AAV2-EYE-08 to AAV2-EYE-22 mutant viruses prepared by the scheme of the present invention in the test examples of the present invention. Figure 7 is an in vivo imaging diagram after injection of fluorescent substrate, and Figure 8 is a chemiluminescence detection result diagram of ocular tissue.
Figure 9 is a Cap 561-588 amino acid sequence alignment diagram of wild-type AAV2, AAV2-7M8 and AAV2-EYE-08 to AAV2-EYE-22 mutant sequences prepared by the scheme of the present invention in the test examples of the present invention.

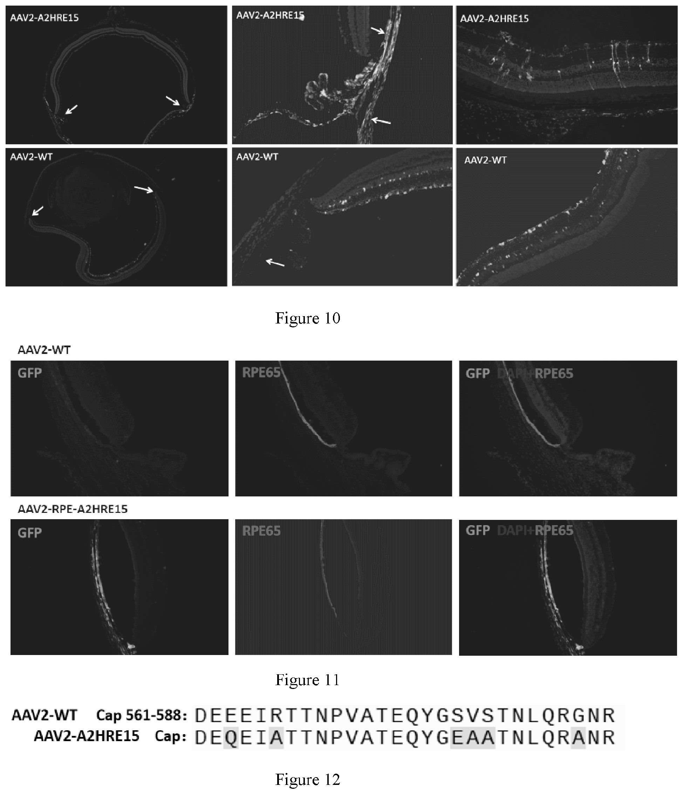

Figures 10-11 are ocular tissue infection effect diagrams of mouse eyes infected for 3 weeks with wild-type AAV2 and AAV2-A2HRE15 mutant viruses prepared by the scheme of the present invention in the test examples of the present invention. Figure 10 is a fluorescence photograph of frozen sections of ocular tissue, and Figure 11 is a fluorescence photograph after immunofluorescence staining.
Figure 12 is a Cap 561-588 amino acid sequence alignment diagram of wild-type AAV2 and AAV2-A2HRE15 mutant sequences prepared by the scheme of the present invention in the test examples of the present invention.

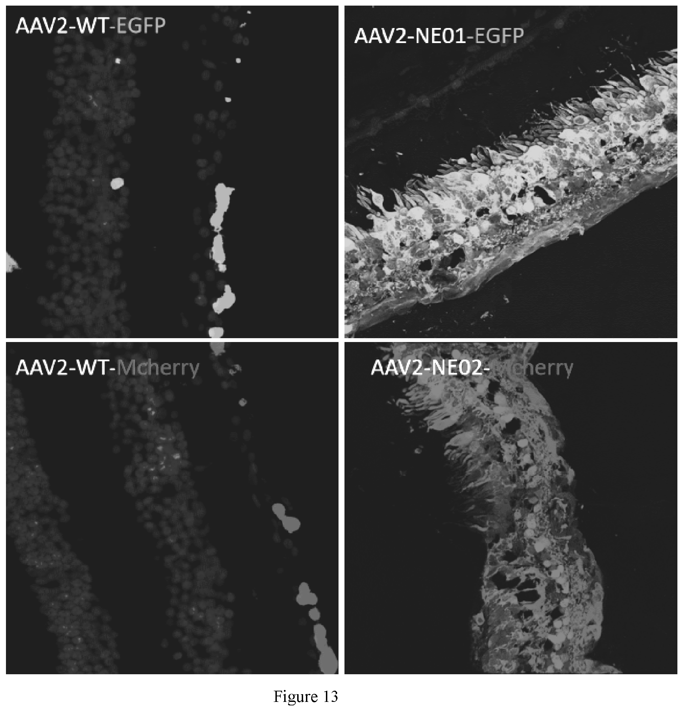

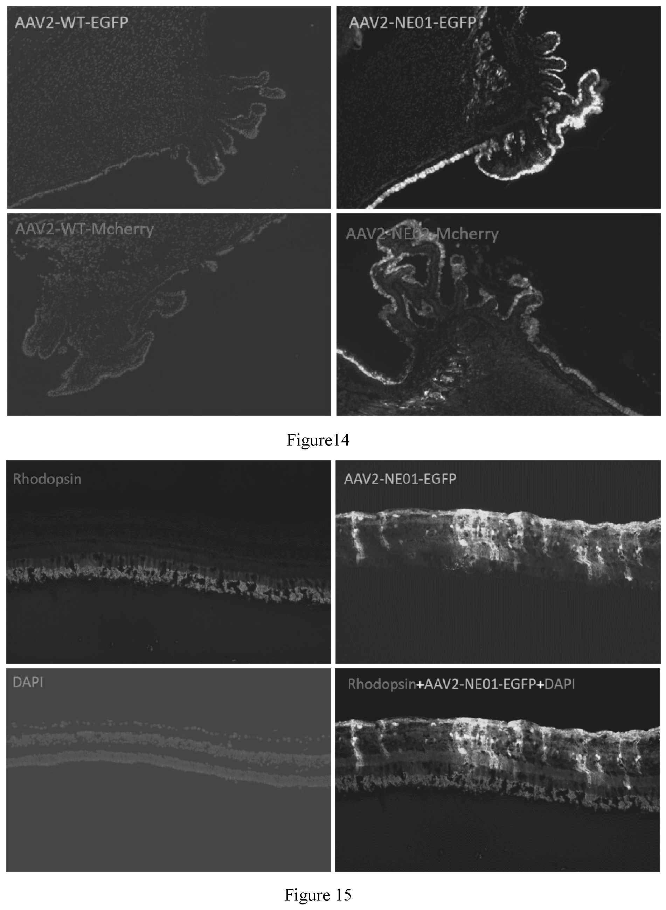

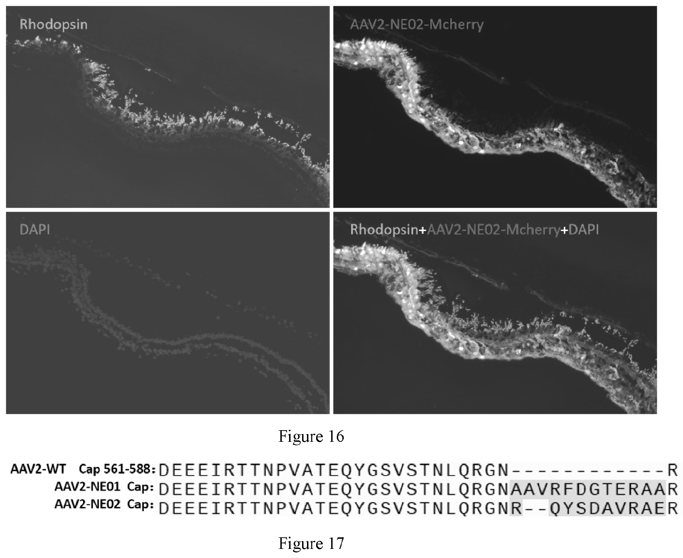

Figures 13-16 are ocular tissue infection effect diagrams of cynomolgus monkey ocular tissue infected for 3 weeks with wild-type AAV2, AAV2-NE01 and AAV2-NE02 viruses prepared by the scheme of the present invention in the test examples of the present invention. Figure 13 is a fluorescence photograph of retinal region infection effect, Figure 14 is a fluorescence photograph of corneal limbus region infection effect, Figure 15 is a fluorescence photograph after immunofluorescence staining of retina infected with AAV2-EGFP and AAV2-NE01-EGFP, and Figure 16 is a fluorescence photograph after immunofluorescence staining of retina infected with AAV2-mCherry and AAV2-NE02-mCherry.
Figure 17 is a Cap 561-588 amino acid sequence alignment diagram of wild-type AAV2, AAV2-NE01 and AAV2-NE02 mutant sequences prepared by the scheme of the present invention in the test examples of the present invention.

### Embodiments of the Present Invention

As used herein, "vector" refers to a macromolecule or macromolecular association that comprises or is associated with a polynucleotide molecule and can be used to mediate delivery of the polynucleotide molecule to a cell. Exemplary vectors include, for example, plasmids, viral vectors, liposomes and other gene delivery vehicles.

The term "AAV" is an abbreviation for adeno-associated virus and can be used to refer to the virus itself or derivatives thereof. Unless otherwise specified, the term encompasses all subtypes as well as naturally occurring and recombinant forms. AAV variants generally refer to mutations at one or several amino acid positions based on a certain wild-type serotype AAV, such as AAV2, AAV9, etc., such as deletion mutations, point mutations, insertion mutations, etc. The biological effects of AAV variants, such as viral yield, infection ability, immunogenicity, etc., differ from the original AAV to a certain extent. The newly mutated amino acid sequence itself may have specific effects, such as the common Y-F mutation, which can reduce the possibility of AAV being degraded by proteasomes and improve AAV infection effect (see Zhong L, Li B, Mah C S, et al. Next generation of adeno-associated virus 2 vectors: point mutations in tyrosines lead to high-efficiency transduction at lower doses[J]. Proceedings of the National Academy of Sciences, 2008, 105(22): 7827-7832), and this characteristic is effective in different serotypes such as AAV2, AAV8, and AAV9 (see Petrs-Silva H, Dinculescu A, Li Q, et al. High-efficiency transduction of the mouse retina by tyrosine-mutant AAV serotype vectors[J]. Molecular therapy, 2009, 17(3): 463-471). The specificity of AAV vectors is mainly determined by their capsid polypeptides. The same capsid polypeptide often exhibits the same or similar functions in different AAV vectors, and capsid polypeptides can self-assemble with other AAV structural proteins to form complete capsids and package genes.

"AAV particle" or "AAV particle" refers to a viral particle composed of at least one AAV capsid polypeptide and an encapsidated polynucleotide AAV vector. Therefore, the production of AAV particles necessarily includes the production of AAV vectors, as such vectors are contained within AAV particles.

"Gene" refers to a polynucleotide containing at least one open reading frame capable of encoding a specific gene product after transcription and sometimes translation. The term "gene" or "coding sequence" refers to an in vitro or in vivo nucleotide sequence encoding a gene product. In some cases, a gene consists of or essentially consists of a coding sequence, i.e., a sequence encoding a gene product. In other cases, a gene includes additional non-coding sequences. For example, a gene may or may not include regions before and after the coding region, such as 5' untranslated region (5'UTR) or "leader" sequence, or 3'UTR or "tail" sequence, as well as intervening sequences (introns) between individual coding segments (exons).

As used herein, "expression vector" encompasses vectors, including but not limited to plasmids, minicircles, viral vectors, liposomes, etc. known in the art, and is used to achieve expression of a gene product in an intended target cell, wherein the vector comprises a polynucleotide encoding the gene product of interest.

The sequences, proteins and fragments of the present invention can be produced by any suitable method, including recombinant production, chemical synthesis or other synthetic methods. Such production methods are within the knowledge of those skilled in the art and are not limitations of the present invention.

The following will describe the conception of the present invention and the technical effects produced in a clear and complete manner in conjunction with embodiments, to fully understand the objects, features and effects of the present invention. Obviously, the described embodiments are only part of the embodiments of the present invention, not all embodiments. Based on the embodiments of the present invention, other embodiments obtained by those skilled in the art without creative effort shall fall within the protection scope of the present invention. The test methods used in the embodiments are conventional methods unless otherwise specified; the materials and reagents used, unless otherwise specified, are reagents and materials available from commercial sources.

### Example 1

This embodiment constructs a library based on previous amino acid substitution methods at positions D561 to R588 and polypeptide insertion methods at positions 587 to 588, and underwent multiple rounds of directed evolution. From NGS sequencing data, sequences with excellent whole-eye, retinal infection ability and penetration ability were screened, providing various mutants including AAV2-ROD-01 to AAV2-ROD-19, AAV2-EYE-08 to AAV2-EYE-22, AAV2-A2HRE15, AAV2-NE01, and AAV2-NE02. Information on amino acid sequences replaced at positions D561 to R588 of the Cap protein in different AAV2 mutants is shown in Table 1.

**Table 1. Information on amino acid sequences replaced at positions D561 to R588 of the Cap protein in AAV2 mutants**

| Sequence Name | Cap 561-588 Amino Acid Sequence | SE Q ID NO. |
|---|---|---|
| AAV2-WT | DEEEIRTTNPVATEQYGSVSTNLQRGNR | 4 |
| AAV2-7M8 | DEEEIRTTNPVATEQYGSVSTNLQRGNLALGETTRPAR | 5 |
| AAV2-ROD-01 | DEEEIRQTNPVATEQYGSVCTNLQRPNR | 6 |
| AAV2-ROD-02 | DESEIATVNPVATEQYGAVSTNLQRPNR | 7 |
| AAV2-ROD-03 | DEQEIATTNPVATEQYGVVSTNLQRGNR | 8 |
| AAV2-ROD-05 | DEHEIATTNPVATENYGDCSTNLQRGNR | 9 |
| AAV2-ROD-06 | DEDEIRTTNPVCTEQYGSVATNIQRGNR | 10 |
| AAV2-ROD-07 | DEDEIATTNPVATEQYGSVSTNLQRGNR | 11 |
| AAV2-ROD-08 | DEEEIRTTNPVATEQYGSVSTNLQRGNLAAQLGQKTIAAR | 12 |
| AAV2-ROD-09 | DEEEIRTTNPVATEQYGSVSTNLQRGNASAGLLGGKPAGR | 13 |
| AAV2-ROD-10 | DEEEIRTTNPVATEQYGSVSTNLQRGNTGGQAYGPKGLSR | 14 |
| AAV2-ROD-11 | DEEEIRTTNPVATEQYGSVSTNLQRGNTGTGAVNRGPGLSR | 15 |
| AAV2-ROD-12 | DEEEIRTTNPVATEQYGSVSTNLQRGNTGPATGTSPRGLSR | 16 |
| AAV2-ROD-15 | DEEEIRTTNPVATEQYGSVSTNLQRGNTGGASYGLPGSRGLS R | 17 |
| AAV2-ROD-16 | DEEEIRTTNPVATEQYGSVSTNLQRGNTGSSASLQRGLSR | 18 |
| AAV2-ROD-17 | DEEEIRTTNPVATEQYGSVSTNLQRGNTGSGIKESRGLSR | 19 |
| AAV2-ROD-18 | DEEEIRTTNPVATEQYGSVSTNLQRGNTGWSEKRPQQGLSR | 20 |
| AAV2-ROD-19 | DEEEIRTTNPVATEQYGSVSTNLQRGNTGSSYGVKVGLSR | 21 |
| AAV2-EYE-08 | DEEEIATVNPVATEQYGSVSTNLQRGNR | 22 |
| AAV2-EYE-09 | DENEIATTNPVATEQYGWVSTNLQRGNR | 23 |
| AAV2-EYE-10 | DEEEIVATNPVATEMYGVVSTNLQRGNR | 24 |
| AAV2-EYE-11 | DEQEIAATNPVATEQYGSVSYNLQRINR | 25 |
| AAV2-EYE-12 | DESEIATTNPVATEQYGVVSTNLQRGNR | 26 |
| AAV2-EYE-13 | DEEEIRTTNPVATEQYGSVSTNLQRGNAAAQGYPPKPPAR | 27 |
| AAV2-EYE-14 | DEEEIRTTNPVATEQYGSVSTNLQRGNTGTSQLGARGLSR | 28 |
| AAV2-EYE-15 | DEEEIRTTNPVATEQYGSVSTNLQRGNTGSARGGYTGLSR | 29 |
| AAV2-EYE-16 | DEEEIRTTNPVATEQYGSVSTNLQRGNTGTSTFGASRGLSR | 30 |
| AAV2-EYE-17 | DEEEIRTTNPVATEQYGSVSTNLQRGNTGSVTFSGTQRGLSR | 31 |
| AAV2-EYE-18 | DEEEIRTTNPVATEQYGSVSTNLQRGNTGSALPSHLSGRGLS R | 32 |
| AAV2-EYE-19 | DEEEIRTTNPVATEQYGSVSTNLQRGNTGLQATMGRGLSR | 33 |
| AAV2-EYE-20 | DEEEIRTTNPVATEQYGSVSTNLQRGNTGSLGTNPTRGLSR | 34 |
| AAV2-EYE-21 | DEEEIRTTNPVATEQYGSVSTNLQRGNTGSYVGQPPRGLSR | 35 |
| AAV2-EYE-22 | DEEEIRTTNPVATEQYGSVSTNLQRGNTGRDKVGIQGLSR | 36 |
| AAV2-A2HRE1 5 | DEQEIATTNPVATEQYGEAATNLQRANR | 37 |
| AAV2-NE01 | DEEEIRTTNPVATEQYGSVSTNLQRGNAAVRFDGTERAAR | 38 |
| AAV2-NE02 | DEEEIRTTNPVATEQYGSVSTNLQRGNRQYSDAVRAER | 39 |

The preparation of the adeno-associated virus variants comprises the following steps:
1. Plasmid Construction
   Through Genewiz Biotechnology Co., Ltd., nucleic acid sequences corresponding to amino acid sequences of AAV2-ROD-01 to AAV2-ROD-19, AAV2-EYE-08 to AAV2-EYE-22, AAV2-A2HRE15, AAV2-NE01, and AAV2-NE02 (as shown in Table 1) were synthesized. The synthesized sequences were cloned into the pAAV2-WT plasmid by seamless cloning method, replacing the Cap protein positions 561 to 588 sequence with the mutant sequences shown in Table 1 to construct pAAV2-mutant plasmid vectors. The ligation products were transformed into E. coli competent cells stbl3, single colonies were picked for culture, and plasmids were extracted for sequencing and enzyme digestion verification. Agarose gel electrophoresis confirmed successful construction of the mutant plasmids.
2. AAV2-WT, AAV2-7M8 and Other AAV2-Mutant Virus Packaging pAAV2-WT or pAAV2-mutant packaging plasmids, together with pAAV-CAG-EGFP (or pAAV-CAG-Luciferase or pAAV-CAG-Mcherry) expression plasmids and pHelper auxiliary plasmids, were co-transfected into suspension HEK293T cells. After 72 hours of continued culture, cells were lysed to harvest virus, and virus was purified by iodixanol density gradient centrifugation. The main steps include:
   A. Cell Culture
      (1) A vial of suspension 293T cells was taken from the liquid nitrogen tank and placed in a 37°C water bath. The cells were quickly shaken to thaw, added to a centrifuge tube containing 5 mL Wayne 293 serum-free medium, centrifuged at 200×g for 5 min, the supernatant was discarded, and resuspended in 10 mL Wayne 293 serum-free medium. The cells were placed in a 125 mL triangular shake flask, supplemented with Wayne 293 serum-free medium to 20 mL, and cultured in an oscillating incubator at 120 rpm, 37°C, 5% CO2.
      (2) After 48-72 hours of culture, when the cell density reached 2-3E+6 cells/mL, the cells were passaged.
      (3) Seed cells were taken to a clean bench, shaken well, and approximately 500 µL sample was taken. 20 µL sample was added to 20 µL trypan blue and mixed well. 20 µL of the mixture was added to a cell counting plate, and 3 readings were taken on an automatic cell counter. The average value was the cell density.
      (4) An appropriate amount of cells was taken and diluted with fresh Wayne 293 serum-free medium to 0.6-0.65E+6 cells/mL, placed in a 500 mL triangular shake flask with 25 mL culture volume per flask. Cultured in an oscillating CO2 cell incubator at 120 rpm, 37°C, 5% CO2.
   B. Cell Transfection
      (1) The above cells were cultured for 48 h, and when the cell density reached 3-3.8E+6 cells/mL, the cells could be transfected.
      (2) For each flask of cells, packaging plasmid, pHelper, and pPAAV-CAG-EGFP (or pAAV-CAG-Mcherry) totaling 100 µg were sequentially added to 4 mL DMEM medium and mixed well, which was the "plasmid dilution".
      (3) Another 1 mL DMEM medium was taken, 300 µg PEI was added and mixed, which was the "PEI dilution".
      (4) The "PEI dilution" was poured into the "plasmid dilution", quickly mixed, and allowed to stand at room temperature for 25 minutes to form a transfection complex. The transfection complex was added dropwise to the cell solution while gently shaking.
      (5) The culture flask was placed in an oscillating CO2 cell incubator and cultured at 120 rpm, 37°C, 5% CO2.
   C. Virus Harvest
      (1) After 72 hours of continued culture post-transfection, virus harvest began.
      (2) The cell suspension from each flask was collected in a centrifuge bottle and centrifuged at 1000 g for 5 min. The supernatant was collected in a new centrifuge bottle, and 0.245 mL of 50% PEG8000 solution (containing 0.5 mol/L NaCl) was added per mL of supernatant, mixed well, and allowed to stand overnight at 2-8°C.
      (3) An appropriate amount of 0.5% Triton X-100 cell lysis buffer (containing omnipotent nuclease) was added to the cell pellet, treated at 37°C for 1-2 h, then 1/10 volume of 5 mol/L NaCl was added, mixed well, and centrifuged at 4°C, 3000 g for 15 min. The supernatant was collected as "crude virus extract" and temporarily stored at 2-8°C.
      (4) The supernatant after PEG8000 precipitation was centrifuged at 4°C, 3000 g for 15 min, the supernatant was discarded, and resuspended with the "crude virus extract" from step (3) for later use.
   D. Virus Purification
      1) An Ultra-Clear centrifuge tube was taken, and 0.5 mL 60% iodixanol, 2 mL 40% iodixanol, 1.5 mL 25% iodixanol, 1.5 mL 15% iodixanol, and collected virus suspension were successively added to the bottom, and finally cell lysate was used for balancing.
      2) Ultracentrifugation at 10°C, 230,000 g, acceleration 8 deceleration 9 for 18 h.
      3) An ultrafiltration tube was taken, and the filter membrane was wetted with 1 mL PBS buffer.
      4) The 40% iodixanol-60% iodixanol interlayer from the ultracentrifuge tube was carefully extracted with a pipette, avoiding protein aspiration, and transferred to the ultrafiltration tube.
      5) An appropriate amount of PBS buffer was added, pipetted uniformly, and centrifuged at 4500 g for 3-5 min. This step was repeated 5-7 times until iodixanol was removed.
      6) The crude virus product from step 5 was diluted to 8 mL, and steps 1 to 5 were repeated.
      7) 1 mL PBS buffer was added and pipetted 40-50 times to form a virus suspension, which was transferred to an EP tube.
      8) A 5 mL syringe was used to aspirate the virus suspension from the EP tube, filtered through a 0.22 µm filter, 20 µL of virus solution was collected as a detection sample, and then aliquoted at 100 µL per tube to obtain adeno-associated virus variants.
3. Virus Titer Detection
   (1) Virus Lysis
      1) 20 µL of virus samples from Example 1 and comparative examples were respectively taken.
      2) 1 µL each of 10% SDS, 0.5 mol/L EDTA, and proteinase K were respectively added and mixed.
      3) Incubated at 56°C in a thermomixer for 1 hour, then incubated at 90°C for 10 min.
      4) 10 µL of virus lysate was taken and diluted 10-fold in gradient to 10,000-fold, which was the virus dilution, for later use.
   (2) Preparation of Standards for Standard Curve
      The plasmid standard with original concentration of 2×10^12 copies/mL was diluted with ultrapure water ddH2O in proportion to 6 gradients as templates for standards: 2×10^11 copies/mL, 2×10^10 copies/mL, 2×10^9 copies/mL, 2×10^8 copies/mL, 2×10^7 copies/mL, 2×10^6 copies/mL.
   (3) Absolute Quantification qPCR
      1) 0.2 mL PCR tubes were taken, and the following reaction system was prepared, with 3 replicates for each virus dilution: 2× qPCR Mix 10 µL; forward and reverse primers 0.2 µL each; 5 µL virus dilution; 4.2 µL ddH2O.
         Amplification primers:
         Forward primer 5'-GGAACCCCTAGTGATGGAGTT-3' (SEQ ID NO: 2);
         Reverse primer 5'-CGGCCTCAGTGAGCGA-3' (SEQ ID NO: 3);
      2) qPCR amplification conditions
         Pre-denaturation: 95°C, 2 min;
         40 cycles: 95°C, 15 s; 60°C, 60 s;
      3) qPCR data processing:
         Virus titer = dilution factor × viral genome copy number.

### Comparative Example 1

This comparative example provides a wild-type adeno-associated virus (AAV2-WT). The AAV2-mutant was prepared according to the method of Example 1, with the only difference being that the adeno-associated virus capsid polypeptide sequence used the wild-type AAV2 capsid polypeptide sequence.

### Comparative Example 2

This comparative example provides an AAV2 adeno-associated virus mutant (AAV2-7M8) that has been used in ophthalmic clinical trials and has strong ocular tissue targeting. The AAV2-mutant was prepared according to the method of Example 1, with the only difference being that the adeno-associated virus capsid polypeptide sequence used the mutant AAV2-7M8 capsid polypeptide sequence (SEQ ID NO: 1).

### Test Example 1: Mouse Retinal Infection Effect Test

(1) Intravitreal Virus Injection
   CAG-EGFP virus samples prepared from the examples and comparative examples were respectively diluted to 2E+9 vg/µL and intravitreally injected into C57BL-6J mice, with 1.5 µL injected per eye (total virus amount of 3E+9 vg), 3 mice per virus. The specific operation steps were as follows:
   1) Before injection anesthesia, ophthalmic examination (slit lamp) was performed to observe the ocular surface condition. If abnormalities were found, the mouse was excluded to ensure there was no ocular disease.
   2) After anesthetizing the mouse, tropicamide was instilled into the eyeball for pupil dilation. After mydriasis, tetracaine hydrochloride was instilled for ocular surface anesthesia.
   3) An appropriate amount of virus was aspirated with a micro-syringe for later use. The anesthetized mouse was placed on the operating table, and the eye was given lidocaine hydrochloride for local anesthesia. The head was held with the left hand to make the eyeball protrude partially. The right hand held the micro-syringe and inserted it into the vitreous cavity from the posterior corneal margin. The needle tip first entered vertically, then tilted, slowly pushing the drug. After injection, the needle was slowly withdrawn.
(2) Fluorescence Fundus Examination
   3 weeks after virus injection, the mouse was anesthetized and the eye was dilated, then transferred to the observation table. The Micron IV small animal retinal imaging system was used for fluorescence fundus imaging (OCT). The acquisition software was used to generate bright-field fundus images and corresponding fluorescence channel fundus images. Imaging parameters were: Gain (dB) -10 dB, Frame Rate (fps) -2 fps, Exposure-Auto.
(3) Eyeball Frozen Section Preparation and Fluorescence Signal Observation Mice with strong fundus fluorescence signals were selected, euthanized, and eyeballs were extracted to prepare frozen sections. GFP fluorescence signal expression in various regions of ocular tissue was observed. The steps were as follows:
   1) Fresh eyeball tissue was immediately dissected and placed in tissue fixative for fixation for more than 24 h.
   2) After removing the tissue from the fixative, surrounding tissue was trimmed flat. The trimmed tissue block was placed in 10% sucrose solution for dehydration at 4°C refrigerator. After the tissue block sank to the bottom, it was transferred to 20% sucrose solution for dehydration at 4°C refrigerator. After the tissue block sank to the bottom, it was transferred to 30% sucrose solution for dehydration at 4°C refrigerator until it sank to the bottom.
   3) The dehydrated tissue was taken out, surface moisture was absorbed with filter paper, and the cut surface was placed upward in an embedding frame. OCT embedding agent was dropped (avoiding bubbles). The embedding frame was placed in a -20°C refrigerator for freezing for half an hour, then transferred to a - 80°C refrigerator for storage.
   4) Before sectioning, the OCT embedding block was taken out from -80°C and placed in the cryostat chamber to equilibrate temperature for 30 min. Sections were taken according to experimental requirements, and the retained sections were labeled and stored at -20°C for later use.
   5) DAPI prepared at a certain ratio was dropped on the sections and incubated at room temperature in the dark for 3 min. The slides were placed in PBS and washed 3 times, 5 min each time. Anti-fluorescence quenching mounting medium was used for mounting.
   6) Observed with a fluorescence microscope, and images were collected.

The scheme of the present invention intravitreally injected AAV2-WT, AAV2-7M8 and some variant viruses into mice. 3 weeks after virus injection, in vivo fluorescence imaging was performed. Some embodiments and comparative examples were selected. As shown in Figure 1, it can be seen from the figure that the expression of multiple preferred variant viruses such as AAV2-ROD-01, AAV2-ROD-02, and AAV2-ROD-03 in the whole eye was significantly stronger than that of AAV2-WT virus. Fluorescence intensity was analyzed and statistically quantified using ImageJ software, normalized with AAV2 as control, and the fold of fundus fluorescence signal of other variants relative to AAV2 was obtained. The results are shown in Figure 2. Among the selected mutants, 16 variants had GFP expression intensity stronger than AAV2-WT, among which AAV2-ROD-05, AAV2-ROD-09, AAV2-ROD-16, and AAV2-ROD-19 were the strongest, approximately 10 times that of AAV2-WT. The EGFP expression intensity of Comparative Example 2 (AAV2-7M8) was similar to that of AAV2-WT virus.

To investigate the infection efficiency on various layers of the retina, after DAPI staining of paraffin sections of eyeballs, fluorescence microscope observation results showed that after intravitreal injection of variant viruses, they could infect various cell layers of the retina from RGC to PRC, while AAV2-WT mainly infected RGC layer cells. The infection area and ability to infect retinal posterior layer cells of variant viruses were stronger than AAV2-WT. Some embodiments and comparative examples were selected as shown in Figure 3. Multiple preferred variants such as AAV2-ROD-01, AAV2-ROD-02, and AAV2-ROD-03 had larger infection ranges than AAV2-WT and 7M8. From the perspective of infection layers, AAV2-WT mainly infected RGC layer cells. 7M8 had certain penetration ability and could penetrate to the posterior layer of the retina to infect a small amount of PRC cells. Multiple preferred variants such as AAV2-ROD-01, AAV2-ROD-02, and AAV2-ROD-03 could infect various cell layers of the retina from RGC to PRC, among which AAV2-ROD-05 had the best infection rate and expression effect on PRC cells.

The retinal region and retinal posterior layer region were respectively selected using ImageJ software, and corresponding region fluorescence intensity was quantified. The retinal region was normalized with AAV2-WT as control, and the fold of whole retinal expression of other variants relative to AAV2-WT was obtained. The results are shown in Figure 4. AAV2-ROD-10 had the strongest whole retinal infection ability, 10 times that of AAV2-WT, and other variants were 3 to 5.5 times that of AAV2-WT. The retinal posterior layer region was normalized with AAV2-7M8 as control, and the fold of penetration ability of other variants relative to 7M8 was obtained. The results are shown in Figure 5. AAV2-WT could not infect retinal posterior layer cells. AAV2-ROD-05 had the strongest penetration ability, 2.1 times that of AAV2-7M8, and other variants were 1.1 to 1.7 times that of AAV2-7M8. This proves that the adeno-associated virus variants prepared by the technology of the present invention have significantly improved infection ability for the whole retina and retinal posterior layer.

The sequence alignment result diagram of adeno-associated virus variant capsid polypeptides is shown in Figure 6. The difference between the sequences of the ROD-01 to ROD-19 capsid polypeptides of the adeno-associated virus variants and the wild-type AAV2 capsid polypeptide is that the sequence of the wild-type capsid polypeptide D561 to R588 (shown in SEQ ID NO: 4) was replaced with polypeptide sequences of 28 to 42 amino acids. Among them, ROD-01 to ROD-07 were partial amino acid substitutions, and ROD-08 to ROD-19 were multiple amino acid insertions.

### Test Example 2: Mouse Whole Eye Expression Ability Test

CAG-Luciferase virus prepared from the examples and comparative examples was respectively diluted to 2E+9 vg/µL and intravitreally injected into Bab/c mice, with 1.5 µL injected per eye (total virus amount of 3E+9 vg), 3 mice per virus. Luciferase expression was detected by in vivo imaging and chemiluminescence methods 3-4 weeks after injection.

### (1) In Vivo Imaging Detection

3 weeks after virus injection, D-luciferin potassium salt solution was intraperitoneally injected. 2 min after injection, the mouse was placed in the induction chamber of the anesthesia machine for isoflurane induction anesthesia. When the mouse's breathing stabilized and there were no large movements, it was transferred to the breathing mask of the imaging instrument for maintenance anesthesia. Imaging parameters were set and imaging was performed.

### (2) Chemiluminescence Detection

### 1) Sample Collection

Mice were euthanized by cervical dislocation, eyelids were opened, and eyeballs were extracted with forceps, removing muscles and connective tissue around the eyeballs. Eyeballs were placed in 2 mL grinding tubes, one eyeball per tube, and temporarily stored on ice.

### 2) Sample Pretreatment

500 µL of 1× Cell Culture Lysis Reagent was added to the homogenization tube containing mouse eyeballs (single eyeball), placed in a grinder pre-cooled to -65°C for homogenization. After homogenization, incubated at 4°C for 1-2 h, centrifuged at 15,000 r/min at 4°C for 3 min, and the supernatant was aspirated.

### 3) Color Development

A black opaque 96-well plate was taken, 20 µL of sample was added to each well, 2-3 replicate wells per sample. Substrate color development solution reconstituted at room temperature in the dark was taken out, and 100 µL was added to each well. Immediately after adding the color development solution, it was placed in a fluorescence microplate reader for detection, and fluorescence signal values were read.

The scheme of the present invention intravitreally injected AAV2-WT, AAV2-7M8 and variant viruses into mice. In vivo imaging results 3 weeks later showed that all mice injected with 15 preferred mutant viruses such as AAV2-EYE-08 to AAV2-EYE-22 had ocular Luciferase signals stronger than AAV2-WT and AAV2-7M8. Some embodiments and comparative examples were selected as shown in Figure 7, indicating that the adeno-associated virus variants prepared by the scheme of the present invention can improve the infection ability of AAV2 virus for the whole eye.

To more accurately quantify the expression effect, eyeballs were extracted and homogenized for chemiluminescence detection. The results are shown in Figure 8. All mice injected with 15 preferred mutant viruses such as AAV2-EYE-08 to AAV2-EYE-22 had whole eye Luciferase expression higher than AAV2-WT and AAV2-7M8, 1.23 to 8.4 times that of AAV2-WT. Among them, 15 groups had expression more than 5 times that of AAV2-WT, which was considered to have significantly improved whole eye expression ability, among which AAV2-EYE-09 had the highest expression. This indicates that the adeno-associated virus variants prepared by the scheme of the present invention can improve the whole eye expression ability of AAV2 virus.

The sequence alignment result diagram of adeno-associated virus variant capsid polypeptides is shown in Figure 9. The difference between the sequences of the EYE-08 to EYE-22 capsid polypeptides of the adeno-associated virus variants and the wild-type AAV2 capsid polypeptide is that the sequence of the wild-type capsid polypeptide D561 to R588 (shown in SEQ ID NO: 4) was replaced with polypeptide sequences of 28 to 42 amino acids. Among them, EYE-08 to EYE-12 were partial amino acid substitutions, and EYE-13 to EYE-22 were multiple amino acid insertions.

### Test Example 3: Mouse Ocular Tissue Infection Effect Test

(1) Intravitreal Virus Injection
   CAG-EGFP virus prepared from the examples and Comparative Example 1 was respectively diluted to 2E+9 vg/µL and intravitreally injected into C57BL-6J mice, with 1.5 µL injected per eye (total virus amount of 3E+9 vg), 3 mice per virus. The specific operation procedures and steps were the same as in Test Example 1.
(2) Eyeball Frozen Section Preparation and Fluorescence Signal Observation The specific operation procedures and steps were the same as in Test Example 1.
(3) Immunofluorescence Staining
   Frozen sections were labeled with Anti-RPE65 antibody, and GFP and RPE colocalization was observed under a fluorescence microscope. The specific steps were as follows:
   1) Frozen sections were taken out from the -20°C refrigerator and restored to room temperature and dried. If it was an unfixed sample, it was fixed with tissue fixative for 15 min, then rinsed with running water.
   2) Sections were placed in repair solution, boiled in an autoclave for 3 min with internal steam release. After natural cooling, slides were placed in PBS and washed 3 times, 5 min each time.
   3) Sections were placed in 0.3% Triton X-100 solution and incubated at room temperature in the dark for 10 min. Slides were placed in PBS and washed 3 times, 5 min each time.
   4) Blocking serum was dropped evenly in the immunohistochemistry circle to cover the tissue, and blocked at room temperature for 60 min.
   5) The blocking solution was gently shaken off. Primary antibody prepared at a certain ratio (Anti-RPE65 antibody, abcam, ab231782, 1:250) was dropped on the sections. Sections were placed flat in a wet box and incubated overnight at 4°C or for 1 hour at room temperature.
   6) Slides were placed in PBS and washed 3 times, 5 min each time. After slightly shaking dry, secondary antibody (fluorescently labeled) corresponding to the species of the primary antibody was dropped in the circle to cover the tissue and incubated at room temperature for 60 min. Slides were placed in PBS and washed 3 times, 5 min each time. Slides were placed in PBS and washed 3 times, 5 min each time.
   7) DAPI prepared at a certain ratio was dropped on the sections and incubated at room temperature in the dark for 3 min. Slides were placed in PBS and washed 3 times, 5 min each time.
   8) Anti-fluorescence quenching mounting medium was used for mounting.
   9) Fluorescence signals were observed under a fluorescence microscope.

After DAPI staining of frozen sections of eyeballs, the results are shown in Figure 10. After intravitreal injection, AAV2-A2HRE15 virus could better infect the corneal limbus, trabecular meshwork and RPE, with high infection positive rate and strong fluorescence intensity, and could also infect various cell layers of the retina from RGC to PRC in small amounts. In contrast, AAV2-WT mainly infected RGC layer cells, and its infection ability for RGC layer cells was superior to AAV2-A2HRE15, but it had almost no infection of the corneal limbus, trabecular meshwork and RPE. This proves that the adeno-associated virus variant AAV2-A2HRE15 prepared by the scheme of the present invention has significantly improved infection ability for the corneal limbus, trabecular meshwork and RPE.

To further confirm that AAV2-A2HRE15 can effectively infect RPE cells, after labeling and staining frozen sections of eyeballs with Anti-RPE65 (RPE-specific antibody), the results are shown in Figure 11. Some GFP fluorescence signals could overlap and colocalize with red fluorescence signals (Anti-RPE65), proving that GFP is expressed in RPE cells.

The sequence alignment result diagram of the adeno-associated virus variant capsid polypeptide AAV2-A2HRE15 and the wild-type AAV2 capsid polypeptide is shown in Figure 12. The difference between the sequence of the adeno-associated virus variant capsid polypeptide AAV2-A2HRE15 and the wild-type AAV2 capsid polypeptide (Protein ID: YP_680426.1) is that amino acids 561-588 of the wild-type AAV2 virus capsid polypeptide were replaced with the polypeptide sequence DEQEIATTNPVATEQYGEAATNLQRANR. The specific 6 mutation sites are: E563Q, R566A, S576E, V578A, S579A, G586A.

### Test Example 4: NHP Ocular Tissue Infection Effect Test

AAV2-WT and AAV2-mutant prepared CAG-EGFP or CAG-Mcherry virus were injected into cynomolgus monkeys. 3 weeks later, eyeballs were extracted to prepare frozen sections, and fluorescence signals were directly observed with a microscope. To further determine the layers of retinal cells infected, the retina was subjected to immunofluorescence staining and observed with a microscope.
(1) Intravitreal Virus Injection
   Samples from Example 1 and comparative examples were respectively diluted to 1E+12 vg/µL and intravitreally injected into cynomolgus monkeys, with 100 µL injected per eye (total virus amount of 1E+11 vg), one cynomolgus monkey per virus. The specific operation steps were as follows:
   1) Before injection anesthesia, ophthalmic examination (slit lamp) was performed to observe the ocular surface condition. If abnormalities were found, the monkey was excluded to ensure there was no ocular disease.
   2) After anesthetizing the cynomolgus monkey, tropicamide was instilled into the eyeball for pupil dilation. After mydriasis, tetracaine hydrochloride was instilled for ocular surface anesthesia.
   3) An appropriate amount of virus was aspirated with a micro-syringe for later use. The anesthetized cynomolgus monkey was placed on the operating table, and the eye was given lidocaine hydrochloride for local anesthesia. The head was held with the left hand to make the eyeball protrude partially. The right hand held the micro-syringe and inserted it into the vitreous cavity from the posterior corneal margin. The needle tip first entered vertically, then tilted, slowly pushing the drug. After injection, the needle was slowly withdrawn.
(2) Sample Collection, Frozen Section Preparation and Fluorescence Signal Observation
   3 weeks after virus injection, the cynomolgus monkeys were euthanized, eyeballs were extracted to prepare frozen sections, and GFP fluorescence signal expression in various regions of ocular tissue was observed. The steps were as follows:
   1) Fresh eyeball tissue was immediately dissected and placed in tissue fixative for fixation for 24-48 hours.
   2) After removing the tissue from the fixative, excess surrounding tissue was removed. The eyeball tissue block was cut open with a sharp blade according to experimental requirements. The lens and vitreous body inside were removed. The trimmed eyeball tissue block was subjected to sucrose gradient dehydration at 4°C refrigerator through 10% sucrose-20% sucrose-30% sucrose solution.
   3) The dehydrated tissue was taken out, surface moisture was absorbed with filter paper, and the cut surface was placed upward in an embedding frame. OCT embedding agent was dropped (avoiding bubbles). The embedding frame was placed in a -20°C refrigerator for freezing for half an hour, then transferred to a - 80°C refrigerator for storage.
   4) Before sectioning, the OCT embedding block was taken out from -80°C and placed in the cryostat chamber to equilibrate temperature for 30 min. Sections were taken according to experimental requirements, and the retained sections were labeled and stored at -20°C for later use.
   5) DAPI prepared at a certain ratio was dropped on the sections and incubated at room temperature in the dark for 3 min. The slides were placed in PBS and washed 3 times, 5 min each time. Anti-fluorescence quenching mounting medium was used for mounting.
   6) Images were collected before observation with a fluorescence microscope.
(3) Immunofluorescence Staining
   Frozen sections were respectively labeled with Anti-Rhodopsin antibody, and GFP or Mcherry colocalization with photoreceptor cells was observed under a fluorescence microscope. The specific steps were as follows:
   1) Frozen sections were taken out from the -20°C refrigerator and restored to room temperature and dried. If it was an unfixed sample, it was fixed with tissue fixative for 15 min, then rinsed with running water.
   2) Sections were placed in repair solution, boiled in an autoclave for 3 min with internal steam release. After natural cooling, slides were placed in PBS and washed 3 times, 5 min each time.
   3) Sections were placed in 0.3% Triton X-100 solution and incubated at room temperature in the dark for 10 min. Slides were placed in PBS and washed 3 times, 5 min each time.
   4) Blocking serum was dropped evenly in the immunohistochemistry circle to cover the tissue, and blocked at room temperature for 60 min.
   5) The blocking solution was gently shaken off. Primary antibody prepared at a certain ratio (Anti-Rhodopsin antibody, abcam, ab221664, 1:500) was dropped on the sections. Sections were placed flat in a wet box and incubated overnight at 4°C or for 1 hour at room temperature.
   6) Slides were placed in PBS and washed 3 times, 5 min each time. After slightly shaking dry, secondary antibody (fluorescently labeled) corresponding to the species of the primary antibody was dropped in the circle to cover the tissue and incubated at room temperature for 60 min. Slides were placed in PBS and washed 3 times, 5 min each time. Slides were placed in PBS and washed 3 times, 5 min each time.
   7) DAPI prepared at a certain ratio was dropped on the sections and incubated at room temperature in the dark for 3 min. Slides were placed in PBS and washed 3 times, 5 min each time.
   8) Anti-fluorescence quenching mounting medium was used for mounting.
   9) Fluorescence signals were observed under a fluorescence microscope.

3 weeks after virus injection, samples were collected to prepare frozen sections. After DAPI staining of eyeball sections, observation results under a fluorescence microscope are shown in Figures 13 and 14. After intravitreal injection, AAV2-NE01-CAG-EGFP (green) and AAV2-NE02-CAG-Mcherry (red) viruses showed enhanced infection of various cell layers of NHP retina from the ganglion cell layer (GCL), inner nuclear layer (INL) to outer nuclear layer (ONL) (Figure 13) and the corneal limbus region (Figure 14), with high infection positive rate and strong fluorescence intensity. The infection range and positive rate of AAV2-NE01 were slightly better than those of AAV2-NE02. In contrast, AAV2-WT mainly infected RGC layer cells, but had almost no infection of other layers of NHP retina and trabecular meshwork. This proves that the adeno-associated virus variants AAV2-NE01 and AAV2-NE02 prepared by the scheme of the present invention have significantly improved infection ability for NHP ocular tissue.

To further more accurately locate the layers of retinal cells infected by AAV2-NE01 and AAV2-NE02, eye tissue frozen sections were labeled and stained with Anti-Rhodopsin (photoreceptor outer segment-specific antibody). The results are shown in Figures 15 and 16. It can be seen that GFP signals and mcherry signals overlap with Anti-Rhodopsin signals in the weaker part, and are closely connected in the stronger part. Rhodopsin has weaker expression in the nuclei and inner segments of cone and rod cells, and stronger expression in the outer segments of cone and rod cells, further proving that AAV2-NE01 and AAV2-NE02 have strong retinal penetration ability and can infect the nuclei and inner segments of photoreceptor cells.

The sequence alignment result diagram of the adeno-associated virus variant capsid polypeptides AAV2-NE01 (SEQ ID NO: 38) and AAV2-NE02 (SEQ ID NO: 39) and the wild-type AAV2 capsid polypeptide is shown in Figure 17. The difference between the sequences of the adeno-associated virus variant capsid polypeptides AAV2-NE01 and AAV2-NE02 and the wild-type AAV2 capsid polypeptide (Protein ID: YP_680426.1) is that the polypeptide sequences AAVRFDGTERAA and RQYSDAVRAE were respectively inserted between amino acids 587 and 588 of the wild-type AAV2 virus capsid polypeptide.

In summary, the adeno-associated virus variants prepared by the scheme of the present invention can significantly enhance the ocular tissue-targeted infection performance of AAV2 virus compared with the wild-type.

The GenBank accession number of the amino acid sequence of the wild-type adeno-associated virus capsid polypeptide Cap2 is YP_680426.1. The sequence of the adeno-associated virus variant 7M8 is shown in SEQ ID NO: 1.

In summary, the adeno-associated virus variants prepared by the scheme of the present invention can significantly enhance the whole-eye infection ability of AAV2 virus compared with the wild-type.

The amino acid sequence of the AAV2 adeno-associated virus mutant capsid polypeptide Cap2-7M8, SEQ ID NO: 1:

MAADGYLPDWLEDTLSEGIRQWWKLKPGPPPPKPAERHKDDSRGLVLPGYK
YLGPFNGLDKGEPVNEADAAALEHDKAYDRQLDSGDNPYLKYNHADAEFQ
ERLKEDTSFGGNLGRAVFQAKKRVLEPLGLVEEPVKTAPGKKRPVEHSPVEP
DSSSGTGKAGQQPARKRLNFGQTGDADSVPDPQPLGQPPAAPSGLGTNTMA
TGSGAPMADNNEGADGVGNSSGNWHCDSTWMGDRVTTTSTRTWALPTYN
NHLYKQISSQSGASNDNHYFGYSTPWGYFDFNRFHCHFSPRDWQRLINNNW
GFRPKRLNFKLFNIQVKEVTQNDGTTTIANNLTSTVQVFTDSEYQLPYVLGSA
HQGCLPPFPADVFMVPQYGYLTLNNGSQAVGRSSFYCLEYFPSQMLRTGNNF
TFSYTFEDVPFHSSYAHSQSLDRLMNPLIDQYLYYLSRTNTPSGTTTQSRLQFS
QAGASDIRDQSRNWLPGPCYRQQRVSKTSADNNNSEYSWTGATKYHLNGR
DSLVNPGPAMASHKDDEEKFFPQSGVLIFGKQGSEKTNVDIEKVMITDEEEIR
TTNPVATEQYGSVSTNLQRGNLALGETTRPARQAATADVNTQGVLPGMVW
QDRDVYLQGPIWAKIPHTDGHFHPSPLMGGFGLKHPPPQILIKNTPVPANPST
TFSAAKFASFITQYSTGQVSVEIEWELQKENSKRWNPEIQYTSNYNKSINVDF
TVDTNGVYSEPRPIGTRYLTRNL.

The above is a further detailed description of the present invention and should not be regarded as a limitation on the specific implementations of the present invention. For those of ordinary skill in the art to which the present invention pertains, simple deductions or substitutions made without departing from the concept of the present invention shall fall within the protection scope of the present invention.

## Claims

1. A variant adeno-associated virus (AAV) capsid polypeptide capable of increasing the ability of an AAV to infect ocular tissue, wherein the variant AAV capsid polypeptide is obtained by deleting an amino acid sequence of an AAV capsid polypeptide which is homologous to amino acids at positions D561 to R588 of a wild-type AAV2 capsid polypeptide, and inserting one of the polypeptides shown in SEQ ID No.6 ~ SEQ ID No.39 at the deletion site.

2. The variant AAV capsid polypeptide according to claim 1, wherein the AAV capsid polypeptide is a capsid polypeptide of AAV2 or an AAV2 variant.

3. A gene encoding the AAV variant AAV capsid polypeptide according to claim 1 or 2.

4. An expression vector, wherein the expression vector expresses the AAV variant AAV capsid polypeptide according to claim 1 or 2, or contains the gene according to claim 3.

5. The expression vector according to claim 4, wherein the expression vector is selected from plasmids and viral vectors.

6. The expression vector according to claim 5, wherein the viral vector is a recombinant AAV vector obtained by insertion or replacement modification of an AAV vector.

7. The expression vector according to claim 6, wherein the AAV is selected from any one of AAV1, AAV2, AAV3, AAV4, AAV5, AAV6, AAV7, AAV8, AAV9, AAV10 and variants thereof.

8. The expression vector according to any one of claims 4 ~ 6, wherein the expression vector further comprises a nucleic acid molecule encoding a functional gene product.

9. A recombinant AAV, wherein the AAV capsid polypeptide thereof is as described in claim 1 or 2, or is obtained by cleaving the AAV capsid polypeptide described in claim 1 or 2.

10. The recombinant AAV according to claim 9, wherein the AAV is selected from any one of AAV1, AAV2, AAV3, AAV4, AAV5, AAV6, AAV7, AAV8, AAV9, AAV10 and variants thereof.

11. An AAV particle, comprising a capsid and a gene packaged within the capsid, wherein the capsid comprises the AAV capsid polypeptide according to claim 1 or 2, or the capsid is assembled from capsid polypeptides VP1, VP2 and VP3 formed by the variant AAV capsid polypeptide according to claim 1 or 2, or the capsid is assembled from capsid polypeptides VP1, VP2 and VP3 encoded and expressed by the gene according to claim 3.

12. A composition, comprising the expression vector according to any one of claims 4~8 and a pharmaceutically acceptable carrier, or the recombinant AAV according to claim 9 or 10, or the AAV particle according to claim 11.

13. Use of the composition according to claim 11, wherein the use is selected from: 1) preparing a gene therapy preparation or transgene preparation targeting ocular tissue; 2) constructing a transgenic animal with targeted ocular tissue; and 3) preparing a drug or transgene vector targeting ocular tissue.

14. A gene therapy method for treating an ocular disease, comprising:
S1, diagnosing a patient to confirm that the patient suffers from an ocular disease, and
S2, administering a therapeutically effective amount of a gene therapy preparation to the patient, wherein the gene therapy preparation is the recombinant AAV according to claim 9 or 10, and/or the AAV particle according to claim 11, and a nucleic acid molecule packaged in the recombinant AAV and the AAV particle expresses a factor capable of treating the ocular disease.
